# EUROPÄISCHE PATENTSCHRIFT

(11) **EP 1 159 971 B1**
(45) Veröffentlichungstag und Bekanntmachung des Hinweises auf die Patenterteilung: **09.05.2012**
(21) Anmeldenummer: 01112521.8
(22) Anmeldetag: 23.05.2001
(51) Int. Cl.: A61L 2/04, A61L 2/07, A61L 2/26, A23L 3/10, B65B 55/02, B01J 3/04

(54) **Verfahren zum Sterilisieren von Sterilisiergut in flexiblen Folienverpackungen und periodisch arbeitender Autoklav zur Durchführung desselben**
Process for the sterilisation of articles contained in flexible foil packages and periodically working autoclave for carrying out such process
Procédé de sterilisation d'articles contenu dans des emballages en feuilles flexibles et autoclave travaillant périodiquement pour mettre en oeuvre ce procédé

(30) Priorität: 27.05.2000 DE 10026539
(43) Veröffentlichungstag der Anmeldung: 05.12.2001
(73) Patentinhaber: Fresenius Kabi Deutschland GmbH, 61352 Bad Homburg v.d.H. (DE)
(72) Erfinder: Kessler, Barbara, Dr., 61476 Kronberg (DE); Knierbein, Bernd, Dr., 61267 Neu-Anspach (DE); Grüneberg, Manfred, Dr., 61273 Wehrheim (DE)
(74) Vertreter: Richly, Erik

(56) Entgegenhaltungen:
- EP-A- 0 609 087
- EP-A- 0 891 781
- US-A- 3 835 762
- US-A- 3 971 629

## Beschreibung

Die Erfindung betrifft ein Verfahren zum Sterilisieren von mit einer Lösung zur enteralen Ernährung befüllte Standbodenbeutel, sowie einen periodisch arbeitenden Autoklaven zur Durchführung dieses Verfahrens.

Zur Sterilisation finden Autoklaven Verwendung, in denen der Sterilisiervorgang in drei Phasen abläuft, nämlich dem Steigen, Halten und Fallen. Bekannt sind periodisch und kontinuierlich arbeitende Autoklaven, die sich in der Arbeitsweise und Bauart unterscheiden.

Periodisch arbeitende Autoklaven werden mit einer Charge beschickt und verschlossen, worauf die drei Phasen des Sterilisationsprozesses nacheinander durchlaufen werden. Mit einer neuen Charge kann erst nach dem Entleeren des Autoklaven begonnen werden. Zusätzlicher Zeitaufwand durch die Totzeiten während des Entleerens und Beschickens erweisen sich bei dem periodisch arbeitenden Autoklaven als nachteilig. Ein Vorteil liegt jedoch in den Variationsmöglichkeiten der Sterilisierbedingungen von Charge zu Charge sowie der freien Wahl der Ablaufzeiten. Darüber hinaus ist ein höherer Stützdruck leichter realisierbar.

Die kontinuierlich arbeitenden Autoklaven sind hingegen bezüglich ihres automatischen Prozeßablaufes, der genauen Einhaltung der Sterilisationsbedingungen sowie ihrer Wirtschaftlichkeit den periodisch arbeitenden Autoklaven überlegen.

Die DE-A-197 30 901 beschreibt einen kontinuierlich arbeitenden Autoklaven zum Sterilisieren von in flexiblen Folienverpackungen befindlichem Sterilisiergut. Die Folienverpackungen werden durch den Autoklaven in Stützeinrichtungen gefördert, die fortlaufend gekippt und/oder gedreht werden. Die Stützeinrichtungen weisen eine Boden- und Deckplatte auf, zwischen denen die Folienverpackungen klemmend fixiert sind.

Obwohl die hydrostatischen Autoklaven hinsichtlich des automatischen Prozeßablaufes und der Wirtschaftlichkeit große Vorteile bieten, haben sie für die Sterilisation von in flexiblen Folienverpackungen befindlichem Sterilisiergut aber bisher noch keine breite Anwendung gefunden. Zur Sterilisation werden vor allem die periodisch arbeitenden Autoklaven verwendet, in deren Druckbehälter ein die Folienverpackungen abstützender Stützdruck erzeugt werden kann.

In den bekannten Autoklaven hat sich insbesondere für Produkte mit schlechter Wärmeleitung und größerer Hitzeempfindlichkeit als vorteilhaft erwiesen, wenn die das Produkt enthaltende Verpackung während der Sterilisation bewegt wird. Durch die Bewegung des Sterilisiergutes lassen sich kürzere Sterilisationszeiten erreichen sowie lokale Überhitzungen und damit Anbackungen vermeiden.

Zum Stand der Technik gehören Autoklaven, in denen die Verpackungen rotieren. Aufgrund von Abschattungen ist eine optimale Temperaturverteilung nicht immer gegeben. Die Drehbewegungen setzen voraus, daß die Verpackungen eingespannt werden. Die Einspannung legt aber eine bestimmte Beutelform fest. Auch sind geringe Beuteltoleranzen notwendig, damit sich die Beutel sicher einspannen lassen. Darüber hinaus sind Abdrücke im Einspannbereich nicht auszuschließen. So besteht bei flexiblen Verpackungen die Gefahr der Faltenbildung. Im übrigen erfordert die Einspannung einen hohen apparativen Aufwand. Daher werden die Rotationsautoklaven in der Praxis nur zur Sterilisation flexibler Gebinde eingesetzt, die den hohen mechanischen Beanspruchungen ohne weiteres widerstehen können.

Neben den Rotationsautoklaven sind auch Autoklaven bekannt, bei denen die Verpackungen einer Schüttelbewegung in Längsrichtung ausgesetzt sind. Mit einer derartigen Schüttelbewegung kann aber eine optimale Temperaturverteilung im Sterilisiergut nicht immer sicher gestellt werden. Schüttelbewegungen haben sich zwar bei der Sterilisation von Flaschen bewährt, bei weichen Verpackungen sind sie aufgrund der ruckartigen Bewegungen jedoch nicht von Vorteil.

Der Erfindung liegt die Aufgabe zugrunde, ein Verfahren zum Sterilisieren von Sterilisiergut in flexiblen Folienverpackungeen anzugeben, bei dem die Verpackungen einerseits geringen mechanischen Beanspruchungen ausgesetzt sind und andererseits eine ausreichend homoge Temperaturverteilung im Sterilisiergut gewährleistet ist. Darüber hinaus ist eine Aufgabe der Erfindung, eine Vorrichtung zur Durchführung des Verfahrens bereitzustellen.

Die Lösung dieser Aufgaben erfolgt erfindungsgemäß mit den im Patentanspruch 1 bzw. 7 angegebenen Merkmalen.

Die das Sterilisiergut enthaltenden Folienverpackungen werden bei dem erfindungsgemäßen Verfahren auf flachen Trays, insbesondere mit glatten Auflageflächen frei liegend in einem periodisch arbeitenden Autoklaven sterilisiert. Daher sind die Verpackungen keinen großen mechanischen Beanspruchungen ausgesetzt. Dabei wird unter einer flachen Auflagefläche auch eine Fläche verstanden die zwar eben, aber an einzelnen Stellen durchbrochen ist. Die homogene Temperaturverteilung im Sterilisiergut wird dadurch erreicht, daß die Folienverpackungen während der Sterilisation fortlaufend aus der horizontalen Lage gekippt und wieder in die horizontale Lage gebracht werden. Dabei werden die frei auf den Auflageflächen aufliegenden Verpackungen seitlich abgestützt.

In Versuchen mit anbackungsgefahrdeten Produkten hat sich gezeigt, daß derartige Schaukelbewegungen für eine gleichmäßige Tempeaturverteilung im Sterilisiergut ausreichend sind. Eine Rotation der Verpackungen zur Verbesserung der inneren Konvektion ist bei vielen dieser Produkte nicht erforderlich.

Mit der Schaukelbewegung entfällt die Notwendigkeit, die Verpackungen zu verspannen, so daß sie nur einer verhältnismäßig geringen mechanischen Beanspruchung ausgesetzt sind. Die auf den Trays liegenden Verpackungen erfahren alle die gleiche Bewegung. Die Verpackungen können sich frei Ausdehnen. Auch können Verpackungen mit unterschiedlichen Formen auf ein und demselben Tray sterilisiert werden. Da eine die Verpackungen abdeckende Verspannung fehlt, können sich die Sterilisationsmedien optimal verteilen, was den konvektiven Wärmeübergang vom Sterilisationsmedium auf die Verpackung verbessert. So lassen sich außerhalb der Trays wegen der fehlenden Rotationskäfige die Dampfdüsen optimal anordnen. Eine ungleichmäßige Wärmeverteilung aufgrund von Abschattungen ist während der Aufheiz- und Haltezeit ausgeschlossen. Durch die Traygestaltung mit der Selbstzentrierung der Verpackungen ergeben sich Vorteile bei der Automatisation.

Die Schaukelbewegungen können mit einem verhältnismäßig geringen apparativen Aufwand realisiert werden. Hierfür ist nur eine einfache und robuste Mechanik erforderlich. Weitere Vereinfachungen sind durch die leichtere Traygestaltung möglich. Damit läßt sich die Automatisation insgesamt vereinfachen. Während der Sterilisation werden die Verpackungen schonend behandelt. Fliehkräfte, wie bei der Rotation treten nicht auf.

Wegen der freien Auflage der Folienverpackungen kommen als Sterilisationsmedium nur Dampf/Luft oder eine Berieselung der Verpackungen in Frage. Vorzugsweise wird das Sterilisiergut während der Aufheiz- und Haltephase zur Erzielung einer besonders gleichmäßigen Temperaturverteilung mit Dampf/Luft behandelt. Zum Abkühlen wird das Sterilisiergut vorzugsweise berieselt.

Die einzelnen Trays, die nur eine Kipp-, aber keine Drehbewegung erfahren, lassen sich einfach übereinander stapeln. Weitere Körbe zur Aufnahme der Trays sind nicht daher erforderlich. Zur Erzielung einer optimalen Temperaturverteilung werden die Trays aus der horizontalen Lage soweit gekippt, daß die durch die nicht vollständige Füllung bedingte Luftblase in den Verpackungen während der Kippbewegung von der einen Seite zu der gegenüberliegenden Seite der Verpackung entlang laufen kann. Vorteilhafterweise werden die Folienverpackungen um 10 bis 30°, vorzugsweise 20° gekippt. Pro Minute sollten mindestens 2 Kippbewegungen, vorzugsweise 10-20 Kippbewegungen ausgeführt werden. Grundsätzlich sind aber auch noch mehr als 20 Kippbewegungen pro Minute möglich.

Damit sich das Sterilisationsmedium im Autoklaven nach allen Seiten verteilen kann, weisen die Anflageflächen der Trays vorteilhafterweise Öffungen auf. Diese dienen insbesondere zum Abfließen des Kühlmediums beim Berieseln der Verpackungen.

Unterhalb der Auflageflächen weisen die Trays vorzugsweise gegenüber der Auflagefläche schräg gestellte Leitflächen auf, auf denen das Kühlmedium abfließen kann. Werden die Trays übereinander gestapelt, kann das Kühlmedium dann kaskadenartig über die schräg gestellten Leitflächen von einer Ebene auf die andere Ebene abgeleitet werden. Die Leitflächen sollten so ausgerichtet sein, daß von einem oberen Tray abfließendes Rühlmedium auf den Folienbeutel fließt, der auf dem jeweils darunter liegenden Tray liegt.

Die Trays sind vorteilhafterweise in mehrere Fächer unterteilt, in denen die Folienverpackungen nebeneinander liegen. Die Unterteilung in die Fächer erfolgt vorzugsweise mit senkrecht zu den Auflageflächen stehenden Anlageflächen, an denen sich die Folienverpackungen seitlich abstützen können.

Die innere Konvektion im Sterilisiergut kann noch dadurch weiter verbessert werden, daß der Stützdruck im Autoklaven während der Sterilisation in Abstimmung auf die Schaukelbewegung vorzugsweise sinusförmig verändert wird. Eine Veränderung des Stätzdrucks hat bei langgestreckten Folienverpackungen eine Deformation quer zu deren Längsachse zur Folge, wodurch das Sterilisiergut zusätzlich bewegt wird. So kann mit einer Erhöhung des Stützdrucks die Folienverpackung zusammengedrückt werden, während eine Verringerung des Stützdrucks dazu führt, daß die Folienverpackungen eine ballige Form annehmen. Die Veränderung des Stützdrucks sollte in Abhängigkeit von der Packungsgröße und dem Kopfraum aber nur innerhalb der Bereiche erfolgen, in denen eine Materialüberlastung durch Überdehnung der Verpackungsfolien ausgeschlossen ist. Bei Verpackungen mit einem Inhalt von 200 bis 2000 ml hat sich eine Veränderung des Stützdruckes von 50 bis 500 mbar als vorteilhaft erwiesen.

Das erfindungsgemäße Verfahren findet insbesondere zur Sterilisation von Standbodenbeuteln Verwendung, die insbesondere mit einer Lösung zur enteralen Ernährung befüllt sind. Die Standbodenbeutel können flach auf die Auflageflächen der Trays aufgelegt werden. Während der Kippbewegungen lassen sich die Standbodenbeutel an ihrem Boden ohne die Gefahr von Beschädigungen seitlich abstützen.

Im folgenden wird unter Bezugnahme auf die Zeichnungen ein Ausführungsbeispiel einer Vorrichtung zur Durchführung des erfindungsgemäßen Verfahrens erläutert.

Es zeigen:
- Figur 1: eine stark vereinfachte schematische Darstellung des diskontinuierlich arbeitenden Autoklaven zum Sterilisieren von Flachbodenbeuteln,
- Figur 2: drei übereinander gestapelte Trays zur Auflage der Flachbodenbeutel,
- Figuren 3a und 3b,: eines der Trays von Figur 2 in der Draufsicht und der Seitenansicht,

- Figur 4: die Hubeinrichtung zum Kippen der Trays des Autoklaven und
- Figuren 5a und 5b: den Standbodenbeutel in horizontaler bzw. schräg gestellter Lage.

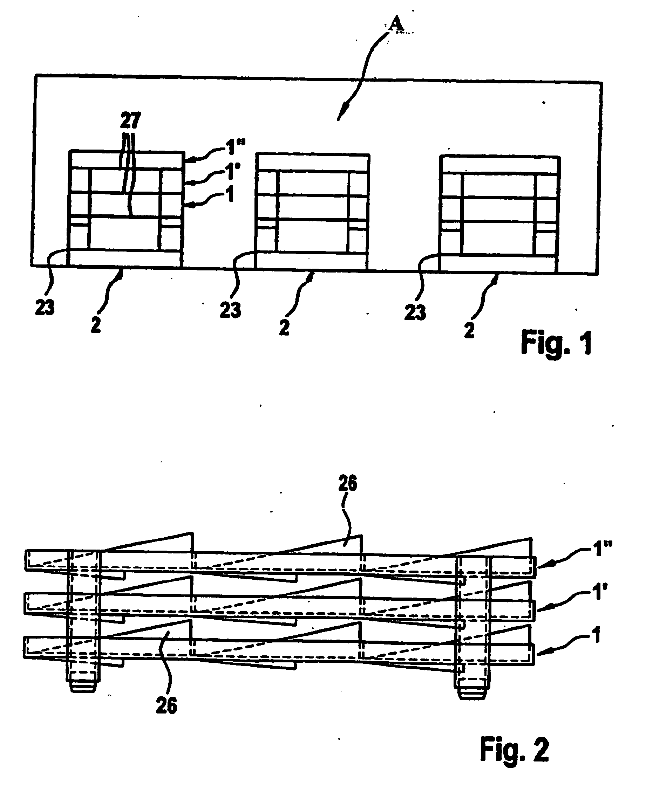

Figur 1 zeigt den Aufbau eines diskontinuierlich arbeitenden Autoklaven in stark vereinfachter schematischer Darstellung. Der Autoklav weist einen rohrförmigen Autoklavenraum A auf, in dem das Sterilisiergut erwärmt, sterilisiert und wieder abgekühlt wird. Der Sterilisierraum A nimmt hintereinander mehrere baugleiche Grundgestelle 2 auf, die jeweils drei übereinander gestapelte Trays 1, 1', 1" tragen. Die Grundgestelle mit den Trays sind in dem Autoklavenraum hintereinander angeordnet. Auf den glatten Auflageflächen 27 der Trays liegen die in Figur 1 nicht dargestellten Standbodenbeutel, die eine Lösung zur enteralen Ernährung enthalten, frei liegend auf.

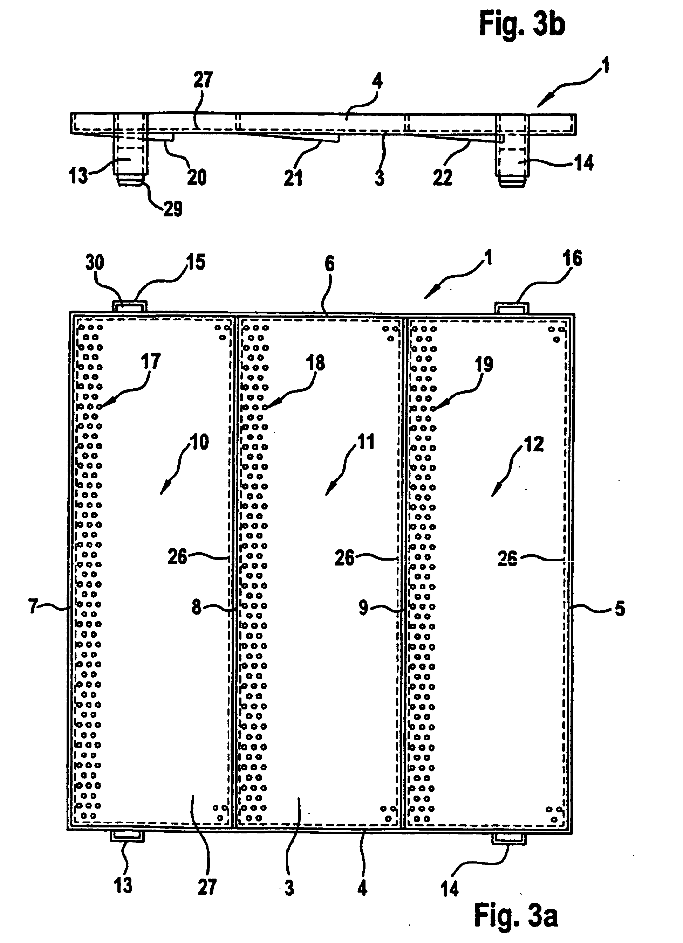

Figur 2 zeigt die drei übereinander gestapelten Trays 1, 1', 1" in der Seitenansicht und die Figuren 3a und 3b eines der Trays in der Draufsicht und der Seitenansicht. Jedes Tray weist eine Bodenplatte 3 aus Edelstahl mit einer glatten Auflagefläche 27 für die Standbodenbeutel und vier Seitenwänden 4, 5, 6, 7 aus Metall auf. Durch zwei Zwischenwände 8, 9 wird jedes Tray in drei Fächer 10, 11, 12 unterteilt. In den Fächern 10, 11, 12 liegen jeweils drei Flachbodenbeutel 26 nebeneinander, deren Umrisse in gestrichelten Linien dargestellt sind. Die Seiten- und Zwischenwände sind Platten aus Metall, die senkrecht auf der Bodenplatte stehen. Alle Teile sind vorzugsweise miteinander verschweißt. Die Trays können aber auch aus sterilisierbeständigen Kunststoffen bestehen.

An den Außenwänden 4, 6 jedes Trays sind jeweils zwei Distanzelemente 13, 14 bzw. 15, 16 befestigt. Die Distanzelemente weisen an ihrem unteren Ende jeweils einen vorspringenden Abschnitt 17 und an ihrem oberen Ende eine entsprechende Ausnehmung 18 auf, so daß sich die einzelnen Trays mit Abstand übereinander stapeln lassen. Die Höhe der Distanzelemente ist derart bemessen, daß ein ausreichender Abstand zwischen der Oberseite des auf dem jeweils unteren Tray aufliegenden Standbodenbeutels und der Bodenplatte des jeweils oberen Trays verbleibt.

Die Zwischenwände 8, 9 und die in den Figuren 2 und 3 auf der rechten Seite liegende Seitenwand 5 bilden Anlageflächen, an denen sich die Standbodenbeutel an ihrem Boden sicher abstützen können. Auf der in den Figuren 2 und 3 gezeigten linken Seite der Fächer 10, 11, 12 weist die Bodenplatte jeweils mehrere Lochreihen 17, 18, 19 auf. An die Unterseite der Bodenplatte 3 sind unterhalb der Fächer 10, 11, 12 sich jeweils bis zur Mitte der Fächer erstreckende Leitbleche 20, 21, 22 angesetzt, die gegenüber der Bodenplatte 3 schräg gestellt sind.

Die übereinander gestapelten Trays 1, 1', 1", die auf den Grundgestellen 2 ruhen, sind gegenüber der horizontalen kippbar. Hierzu ist an jedem Grundgestell eine Hubeinrichtung 23 vorgesehen.

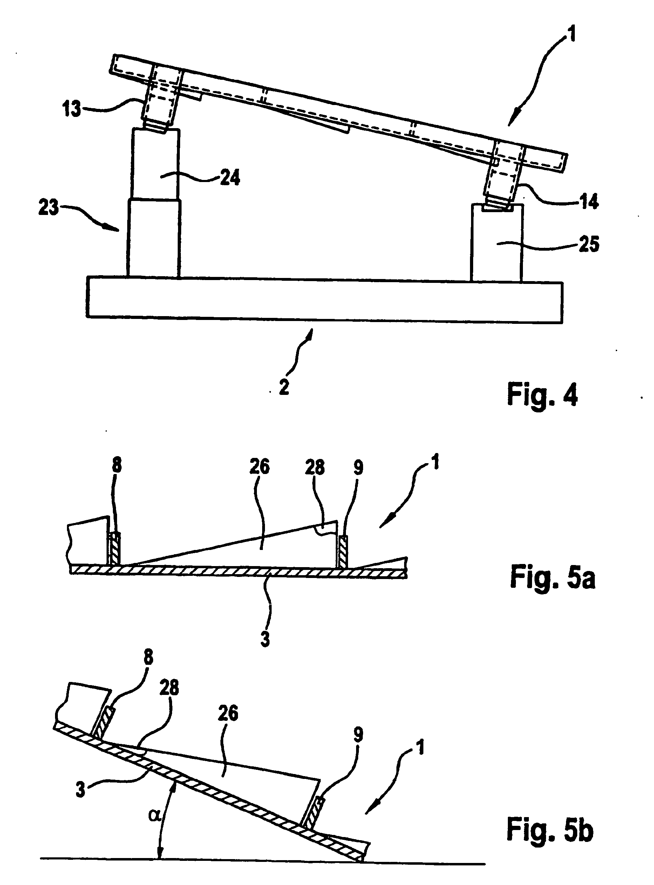

Figur 4 zeigt eines der Grundgestelle 2 zusammen mit der Hubeinrichtung 23 in der Seitenansicht. Die Hubeinrichtung 23 umfaßt insgesamt vier Aufnahmestücke 24, 25, von denen in Figur 4 nur die vorderen sichtbar sind. In die Aufnalunestücke 24, 25 lassen sich die Distanzelemente 13,14,15,16 des untersten Trays 1 einsetzen. Die in Figur 4 auf der linken Seite liegenden Aufnahmestücke 24 sind in horizontaler Richtung verschiebbar geführt, so daß sich die Aufnahmestücke anheben und absenken lassen. Wenn die linken Aufnahmestücke 24 angehoben werden, wird der Tray 1 schräg gestellt. Die Bewegung der Aufnahmestücke kann beispielsweise elektromotorisch, hydraulisch oder pneumatisch erfolgen.

In Figur 4 ist der Tray 1 in der gekippten Position dargestellt. Werden die linken Aufnahmestücke 24 abgesenkt, befindet sich der Tray wieder in der horizontalen Position. Der Winkel, um den der Tray schräg gestellt wird, beträgt ca. 20°. Dieser ist abhängig von der Geometrie der Standbodenbeutel.

Figur 5a zeigt einen auf dem Tray 1 aufliegenden Standbodenbeutel 26 in der horizontalen Lage, während der schräg gestellte Standbodenbeutel in Figur 5b dargestellt ist. In der horizontalen Position befindet sich die aufgrund der nicht vollständigen Füllung vorhandene Luftblase 28 im Beutel am höchsten Punkt, d.h. in Figur 5a auf der rechten Seite. Der Tray wird nun soweit schräg gestellt, daß die Luftblase 28 während der Kippbewegung von der rechten Seite auf die linke Seite läuft (Figur 5b). Dadurch wird während des Sterilisierens die innere Konvektion verbessert und eine homogene Temperaturverteilung erreicht.

Zum Sterilisieren werden die das Sterilisiergut enthaltenden Standbodenbeutel in die Fächer 10,11,12 der Trays 1,1',1" gelegt, die übereinander gestapelt und auf die Hubeinrichtungen 23 der Grundgestelle 2 gesetzt werden. Anschließend wird der Autoklavenraum A mit den Standbodenbeuteln beschickt. In einer Anwärmphase wird das Sterilisiergut in den Standbodenbeuteln mit Dampf/Luft auf die Sterilisiertemperatur erwärmt. Daran schließt sich eine Haltephase an, in der das Sterilisiergut mit Dampf/Luft sterilisiert wird. Die Abkühlung des Sterilisiergutes erfolgt durch Berieseln der Standbodenbeuteln mit einer Kühlflüssigkeit, die unter Druck von oben und seitlich auf die Verpackungen gesprüht wird. Hierzu sind in den Figuren nicht dargestellt Sprühköpfe vorgesehen. Die von den Standbodenbeuteln 26 abfließende Flüssigkeit gelangt durch die Öffnungen 17, 18,19 in der Bodenplatte 3 zu den Leitblechen 20,21,22 , an denen die Flüssigkeit abfließt und zu den Standbodenbeuteln des jeweils unteren Trays gelangt. Mit den Leitblechen wird die Flüssigkeit kaskardenartig durch die einzelnen Ebenen geleitet, so daß alle Beutel ausreichend benetzt werden.

## Patentansprüche

1. Verfahren zum Sterilisieren von Sterilisiergut in flexiblen Folienverpackungen, insbesondere mit einer Lösung zur enteralen Ernährung befüllte Standbodenbeutel, bei dem die aufgrund einer nicht vollständigen Befüllung eine Luftblase enthaltenden Folienverpackungen chargenweise einem Autoklavenraum eines periodisch arbeitenden Autoklaven zugeführt und auf flachen Auflagefläche frei liegend in dem Autoklavenraum sterilisiert werden,
**dadurch gekennzeichnet,**
**dass** die Folienverpackungen während des Sterilisierens fortlaufend aus der horizontalen Lage gekippt und wieder in die horizontale Lage gebracht werden, dass die Luftblase im Kopfraum der Folienverpackungen während der Kippbewegung von der einen zu der gegenüberliegenden Seite der Folienverpackung entlanglaufen kann, wobei die auf den Auflageflächen flach aufliegenden Folienverpackungen während der Kippbewegungen nur an seitlichen Anlageflächen abgestützt werden, ohne klemmend fixiert zu werden.

2. Verfahren nach Anspruch 1, **dadurch gekennzeichnet, dass** die Folienverpackungen um 10 bis 30°, vorzugsweise 20° gekippt werden.

3. Verfahren nach einem der Ansprüche 1 bis 2, **dadurch gekennzeichnet, dass** pro Minute mindestens 2 Kippbewegungen, vorzugsweise 10 bis 20 Kippbewegungen ausgeführt werden.

4. Verfahren nach einem der Ansprüche 1 bis 3, **dadurch gekennzeichnet, dass** die Auflageflächen Öffnungen aufweisen.

5. Verfahren nach einem der Ansprüche 1 bis 4, **dadurch gekennzeichnet, dass** die Folienverpackungen auf mehreren mit Abstand übereinander liegenden Trays in dem Autoklaven sterilisiert werden.

6. Verfahren nach einem der Ansprüche 1 bis 5, **dadurch gekennzeichnet, dass** die Folienverpackungen in einer Dampfphase sterilisiert werden.

7. Verfahren nach einem der Ansprüche 1 bis 6, **dadurch gekennzeichnet, dass** der Stützdruck im Autoklaven zur gezielten Deformation der Folienverpackungen während des Sterilisierens verändert wird.

8. Periodisch arbeitender Autoklav zur Durchführung des Verfahrens nach einem der Ansprüche 1 bis 7, der über einen Autoklavenraum (A) verfügt, in den Trays (1,1',1") mit flachen Auflageflächen zur frei liegenden Auflage der Folienverpackungen eingesetzt sind, auf deren Auflageflächeflexible Folienverpackungen enthaltend Sterilisiergut flach aufliegen, die aufgrund einer nicht vollständigen Befüllung eine Luftblase enthalten,
**dadurch gekennzeichnet,**
**dass** die Trays (1,1',1") fortlaufend aus der horizontalen Lage kippbar und wieder in die horizontalen Lage bringbar sind, dass die Luftblase im Kopfraum der Folienverpackungen während der Kippbewegung von der einen Seite zu der gegenüberliegenden Seite der Folienverpackung entlanglaufen kann, wobei die Trays derart ausbildet sind, dass die auf den Auflageflächen flach aufliegenden Folienverpackungen während der Kippbewegungen nur an seitlichen Anlageflächen abgestützt werden, ohne klemmend fixiert zu werden.

9. Autoklav nach Anspruch 8, **dadurch gekennzeichnet, dass** die Auflageflächen (27) der Trays (1,1',1") Öffnungen (17,18,19) aufweisen.

10. Autoklav nach Anspruch 8 oder 9, **dadurch gekennzeichnet, dass** die Trays (1,1',1") übereinander gestapelt sind.

11. Autoklav nach Anspruch 10, **dadurch gekennzeichnet, dass** die Trays (1,1',1") unterhalb der Auflageflächen (27) angeordnete Leitflächen (20,21,22) aufweisen, die gegenüber den Auflageflächen schräg gestellt sind.

12. Autoklav nach einem der Ansprüche 8 bis 11, **dadurch gekennzeichnet, dass** die Trays (1,1',1") durch senkrecht auf den Auflageflächen stehende Anlageflächen (8,9,5) in mehrere Fächer (10,11,12) unterteilt sind, in die die Folienverpackungen (26) nebeneinander liegend ablegbar sind.

13. Autoklav nach einem der Ansprüche 8 bis 12, **dadurch gekennzeichnet, dass** zum Kippen der Trays (1,1',1") eine an einer Seite der Trays angreifende Hubeinrichtung (23) vorgesehen ist.

## Claims

1. Process for sterilizing a substance to be sterilized in flexible foil packagings, in particular stand-up pouches filled with an enteral feeding solution, in which the foil packagings, containing an air bubble as a result of not being completely filled, are fed in batches to an autoclave chamber of a periodically operating autoclave and are sterilized in the autoclave chamber while lying freely on a flat supporting surface, **characterized in that**, during the sterilization, the foil packagings are continually tilted out of the horizontal position and brought back into the horizontal position and **in that** the air bubble in the space at the top of the foil packagings can run from one side of the foil packaging to the opposite side during the tilting movement, the foil packagings that are lying flat on the supporting surfaces only being supported on lateral bearing surfaces during the tilting movements, without being fixed in a clamping manner.

2. Process according to Claim 1, **characterized in that** the foil packagings are tilted by 10 to 30°, preferably 20°.

3. Process according to either of Claims 1 and 2, **characterized in that** at least 2 tilting movements, preferably 10 to 20 tilting movements, are performed per minute.

4. Process according to one of Claims 1 to 3, **characterized in that** the supporting surfaces have openings.

5. Process according to one of Claims 1 to 4, **characterized in that** the foil packagings are sterilized on a number of trays lying spaced apart one above the other in the autoclave.

6. Process according to one of Claims 1 to 5, **characterized in that** the foil packagings are sterilized in a vapour phase.

7. Process according to one of Claims 1 to 6, **characterized in that** the supporting pressure in the autoclave is varied for specifically intended deformation of the foil packagings during the sterilization.

8. Periodically operating autoclave for carrying out the process according to one of Claims 1 to 7, which has an autoclave chamber (A), in which trays (1, 1', 1'') with flat supporting surfaces are used for supporting the foil packagings in a freely lying manner, on the supporting surface of which trays flexible foil packaging containing a substance to be sterilized lie flat and contain an air bubble as a result of not being completely filled, **characterized in that** the trays (1, 1', 1") can be continually tilted out of the horizontal position and brought back into the horizontal position and **in that** the air bubble in the space at the top of the foil packagings can run from one side of the foil packaging to the opposite side during the tilting movement, the trays being designed in such a way that the foil packagings that are lying flat on the supporting surfaces are only supported on lateral bearing surfaces during the tilting movements, without being fixed in a clamping manner.

9. Autoclave according to Claim 8, **characterized in that** the supporting surfaces (27) of the trays (1, 1', 1") have openings (17, 18, 19).

10. Autoclave according to Claim 8 or 9, **characterized in that** the trays (1, 1', 1") are stacked one above the other.

11. Autoclave according to Claim 10, **characterized in that** the trays (1, 1', 1'') have directing surfaces (20, 21, 22), which are arranged below the supporting surfaces (27) and are set obliquely in relation to the supporting surfaces.

12. Autoclave according to one of Claims 8 to 11, **characterized in that** the trays (1, 1', 1'') are divided by bearing surfaces (8, 9, 5) standing perpendicularly on the supporting surfaces into a number of compartments (10, 11, 12), in which the foil packagings (26) can be placed lying down next to one another.

13. Autoclave according to one of Claims 8 to 12, **characterized in that** a listing device (23) acting on one side of the trays is provided for the tilting of the trays (1, 1', 1 ").

## Revendications

1. Procédé de stérilisation de produits à stériliser dans des emballages en film flexibles, en particulier d'un sac à poser debout rempli d'une solution nutritive entérale, dans lequel les emballages en film contenant une bulle d'air pour cause de remplissage incomplet sont apportés par lots dans un espace d'autoclave d'un autoclave travaillant périodiquement et sont stérilisés en étant posés librement dans l'espace d'autoclave sur une surface plane de pose,
**caractérisé en ce que**
pendant la stérilisation, les emballages en film sont inclinés progressivement hors de la position horizontale et sont ramenés en position horizontale,
**en ce que** la bulle d'air présente dans l'espace de tête des emballages en film peut se déplacer pendant le déplacement d'inclinaison depuis un côté jusqu'au côté opposé de l'emballage en film,
les emballages en film posés à plat sur la surface de pose n'étant soutenus pendant les déplacements d'inclinaison que sur des surfaces latérales d'appui, sans être fixés par serrage.

2. Procédé selon la revendications 1, **caractérisé en ce que** les emballages en film sont inclinés sur 10 à 30° et de préférence sur 20°.

3. Procédé selon l'une des revendications 1 et 2, **caractérisé en ce qu'**au moins 2 déplacements d'inclinaison et de préférence de 10 à 20 déplacements d'inclinaison sont exécutés par minute.

4. Procédé selon l'une des revendications 1 à 3, **caractérisé en ce que** les surfaces de pose présentent des ouvertures.

5. Procédé selon l'une des revendications 1 à 4, **caractérisé en ce que** les emballages en film sont stérilisés dans l'autoclave sur plusieurs plateaux disposés à distance les uns au-dessus des autres.

6. Procédé selon l'une des revendications 1 à 5, **caractérisé en ce que** les emballages en film sont stérilisés dans une phase vapeur.

7. Procédé selon l'une des revendications 1 à 6, **caractérisé en ce que** la pression de soutien dans l'autoclave est modifiée pendant la stérilisation en vue d'obtenir une déformation contrôlée des emballages en film.

8. Autoclave travaillant périodiquement, destiné à exécuter le procédé selon l'une des revendications 1 à 7 et doté d'un espace d'autoclave (A) dans lequel des plateaux (1, 1', 1") à surface de pose plane sont insérées en vue d'y placer des emballages en film qui y sont posés librement, les emballages en film flexibles contenant un produit à stériliser et contenant une bulle d'air pour cause de remplissage incomplet reposant à plat sur les surfaces de pose,
**caractérisé en ce que**
les plateaux (1, 1', 1'") peuvent en permanence être inclinés hors de la position horizontale et ramenés dans la position horizontale,
**en ce que** la bulle d'air présente dans l'espace de tête des emballages en film peut se déplacer d'un côté au côté opposé de l'emballage en film pendant le déplacement d'inclinaison et
**en ce que** les plateaux sont configurés de telle sorte que les emballages en film qui reposent à plat sur les surfaces de pose ne sont soutenus pendant les déplacements d'inclinaison que sur des surfaces latérales d'appui sans être fixés par serrage.

9. Autoclave selon la revendication 8, **caractérisé en ce que** les surfaces de pose (27) des plateaux (1, 1', 1") présentent des ouvertures (17, 18, 19).

10. Autoclave selon la revendication 8 ou 9, **caractérisé en ce que** les plateaux (1, 1', 1") sont empilés les uns au-dessus des autres.

11. Autoclave selon la revendication 10, **caractérisé en ce que** les plateaux (1, 1', 1") présentent en dessous des surfaces de pose (27) des surfaces de guidage (20, 21, 22) qui sont disposées obliquement par rapport aux surfaces de pose.

12. Autoclave selon l'une des revendications 8 à 11, **caractérisé en ce que** les plateaux (1, 1', 1") sont divisés en plusieurs compartiments (10, 11, 12) par des surfaces d'appui (8, 9, 5) placées verticalement sur les surfaces de pose, les emballages en film (26) pouvant y être déposés les uns à côté des autres.

13. Autoclave selon l'une des revendications 8 à 12, **caractérisé en ce qu'**un dispositif de relèvement (23) qui engage les plateaux sur un côté est prévu pour incliner les plateaux (1, 1', 1'').
